(19)

Europäisches
Patentamt

European
Patent Office

Office européen
des brevets

(11) **EP 4 166 002 A1**

(12) **EUROPEAN PATENT APPLICATION**

(43) Date of publication:
**19.04.2023 Bulletin 2023/16**

(21) Application number: **21202048.1**

(22) Date of filing: **12.10.2021**

(51) International Patent Classification (IPC):
**A23K 10/18** (2016.01)    **A23L 33/135** (2016.01)
**A61K 35/744** (2015.01)    **A61K 35/745** (2015.01)
**A61K 35/747** (2015.01)    **C12N 1/04** (2006.01)
**C12N 1/20** (2006.01)

(52) Cooperative Patent Classification (CPC):
**A23L 33/135; A23K 20/147; A23K 20/163;**
**A61K 35/744; A61K 35/745; A61K 35/747;**
**C12N 1/04; C12N 1/20; C12N 1/205;** C12R 2001/01

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Evonik Operations GmbH**
**45128 Essen (DE)**

(72) Inventors:
• **Reus, Christian**
  **63579 Freigericht (DE)**
• **Jäger, Barbara**
  **63533 Mainhausen (DE)**

• **Hochheiser, Ulrike**
  **63594 Hasselroth (DE)**
• **Pelzer, Stefan**
  **33335 Gütersloh (DE)**
• **Borgmann, Cornelia**
  **60486 Frankfurt (DE)**
• **Köstner, Martin**
  **64295 Darmstadt (DE)**

(74) Representative: **Evonik Patent Association**
**c/o Evonik Industries AG**
**IP Management**
**Bau 1042A/PB 15**
**Paul-Baumann-Straße 1**
**45772 Marl (DE)**

(54) **MICROBIAL PREPARATIONS CONTAINING SPECIFIC CRYOPROTECTANTS**

(57)    The present invention concerns microbial preparations which contain a mixture of maltodextrin with a protein hydrolysate as cryoprotectants.

**EP 4 166 002 A1**

**Description**

The present invention concerns microbial preparations which contain a mixture of maltodextrin with a protein hydrolysate as cryoprotectants.

Lactic acid bacteria which are not able to form spores, e.g. bacteria of the species *Enterococcus faecium*, are likely to die under stress conditions like during drying of the fermentation broth or during storage of the biomass or storage of a product containing the bacteria, as, in contrast to spore-forming bacteria, they are not able to switch into a dormant state which significantly improves the ability to survive stress conditions. This results in a reduction of the colony forming units (CFU) per gram of biomass and/or per gram of product which is detrimental to the commercial utilization of such bacteria.

Accordingly, it was an object of the present invention to increase the survival rate of such bacteria under stress conditions. In particular, it was an object of the present invention to provide microbial preparations which allow to increase the survival rate of such bacteria under said stress conditions.

According to the invention it was surprisingly found that the survival rate of the bacteria can be significantly increased, if specific cryoprotectants are added to the bacteria before drying of the biomass. Of the tested cryoprotectants combinations of maltodextrin with protein hydrolysates like peptones and yeast extract turned out to be very useful for solving the object of the invention.

Thus, a first subject of the present invention are microbial preparations containing maltodextrin and at least one protein hydrolysate, wherein the at least one protein hydrolysate is preferably selected from peptones and yeast extract and mixtures thereof.

The microbial preparations according to the invention preferably have a residual moisture content of 0.1 to 6 wt.-%, preferably of 0.3 to 5 wt.-%, more preferably of 0.5 to 4 wt.-%. They further preferably exhibit a water activity ($a_w$ value) of 0.05 to 0.6, preferably of 0.1 to 0.5.

In a preferred embodiment of the invention, the microbial preparations contain 15 to 45 wt.-%, preferably 20 to 40 wt.-%, in particular 25 to 35 wt.-%, maltodextrin and 6 to 24 wt.-%, preferably 10 to 20 wt.-%, in particular 12 to 18 wt.-% of at least one protein hydrolysate, in particular a peptone or a yeast extract or a combination thereof.

The cells to be protected by the cryoprotectants are preferably provided in form of a fermentation broth. Microbial preparations according to the invention thus preferably contain 40 to 75 wt.-%, preferably 45 to 70 wt.-%, in particular 50 to 65 wt.-% of a dried bacterial fermentation broth.

The microbial preparations contain bacterial cells preferably in an amount of $5 \times 10^{10}$ to $5 \times 10^{12}$, more preferably in an amount of $1 \times 10^{11}$ to $4 \times 10^{12}$, in particular in an amount of $5 \times 10^{11}$ to $2 \times 10^{12}$ colony forming units (cfu) per gram.

A further subject of the invention is also a method of preparing the microbial preparations according to the invention, wherein a microbial biomass is mixed with maltodextrin and at least one protein hydrolysate, preferably with a peptone or with a yeast extract or with a combination thereof.

The method for obtaining the microbial preparation preferably comprises the following steps:

   a) Preparation of a bacterial fermentation broth by cultivating bacteria in a fermentation medium and optionally concentrating the fermentation broth,
   b) Addition of maltodextrin and at least one protein hydrolysate, in particular a peptone or a yeast extract or a combination thereof, to the fermentation broth,
   c) Drying of the suspension thus obtained.

The microbial preparations of the invention are preferably used for preparing feed additive compositions by mixing the microbial preparations with suitable carriers.

A further subject of the present invention is therefore also a feed additive containing a microbial preparation according to the invention. The feed additive according to the invention preferably comprises the microbial preparation in an amount of 0.1 to 10 wt.-%, more preferably in an amount of 0.2 to 5 wt.-%, in particular in an amount of 0.3 to 3 wt.-%. The feed additive preferably further comprises a carrier, in particular in an amount of at least 80 wt.-%, preferably in an amount of at least 90 or of at least 95 wt.-%, in particular in an amount of 90 to 99.9 wt.-% or 95 to 99.8 wt.-% or 97 to 99.7 wt.-%. In a preferred embodiment of the invention the feed additive consists only of a mixture of the microbial preparation with said carrier.

The feed additives preferably contain bacteria in an amount of $1 \times 10^9$ to $1 \times 10^{11}$ cfu (colony forming units), more preferably in an amount of $5 \times 10^9$ to $5 \times 10^{10}$ cfu per g feed additive.

The carrier as contained in the feed additive is preferably selected from inert formulation ingredients added to improve recovery, efficacy, or physical properties and/or to aid in packaging and administration. Such carriers may be added individually or in combination. These carriers may be selected from anti-caking agents, anti-oxidation agents, bulking agents, binders, structurants, coatings and/or protectants. Examples of useful carriers include polysaccharides (in particular starches, maltodextrins, methylcelluloses, gums, chitosan and/or inulins), protein sources (in particular skim-milk powder and/or sweet-whey powder), peptides, sugars (in particular lactose, trehalose, sucrose and/or dextrose), lipids (in particular lecithin, vegetable oils and/or mineral oils), salts (in particular sodium chloride, sodium carbonate, calcium carbonate, chalk, limestone, magnesium carbonate, sodium phosphate, calcium phosphate, magnesium phosphate and/or sodium citrate), and silicates (in particular clays, in particular beolite clay, amorphous silica, fumed/precipitated silicas, zeolites, Fuller's earth, Baylith®, clintpolite, montmorillonite, diatomaceous earth, talc, bentonites, and/or silicate salts like aluminium, magnesium and/or calcium silicate). Suitable carriers for animal feed additives are also set forth in the American Feed Control Officials, Inc.' s Official Publication, which publishes annually. See, for example Official Publication of American Feed Control Officials, Sharon Krebs, editor, 2006 edition, ISBN 1-878341-18-9. Preferred carriers according to the invention are selected from calcium carbonate, in particular limestone, dextrose, maltodextrin and combinations thereof.

The feed additives according to the invention preferably have a residual moisture content of 0.1 to 6 wt.-%, preferably of 0.3 to 5 wt.-%, more preferably of 0.5 to 4 wt.-%. They further preferably exhibit a water activity ($a_w$ value) of 0.05 to 0.6, preferably of 0.1 to 0.5.

A further subject of the present invention is also a method of preparing the feed additive according to the invention, wherein the microbial preparation according to the invention is mixed with a carrier, in particular as mentioned above, preferably in amount to adjust an amount of $1 \times 10^9$ to $1 \times 10^{11}$ cfu (colony forming units), more preferably to adjust an amount of $5 \times 10^9$ to $5 \times 10^{10}$ cfu per g feed additive.

Thus, a further subject of the invention is in particular also a method of preparing the feed additive according to the invention, wherein a microbial biomass is mixed with maltodextrin and at least one protein hydrolysate, preferably a peptone or a yeast extract or a combination thereof, and the microbial preparation thus obtained is subsequently mixed with a carrier to obtain the feed additive.

The method for obtaining the feed additive of the invention therefore preferably comprises the following steps:

a) Preparation of a bacterial fermentation broth by cultivating bacteria in a fermentation medium and optionally concentrating the fermentation broth,
b) Addition of maltodextrin and at least one protein hydrolysate, in particular a peptone or a yeast extract or a combination thereof, to the fermentation broth,
c) Drying of the suspension thus obtained,
d) Mixing of the dried suspension with a further carrier, preferably selected from limestone, dextrose and mixtures thereof.

Concentration of the fermentation broth according to step (a) is preferably carried out, if the fermentation broth contains cells in a lower amount. Then, concentration is preferably carried out until a total dry matter (TDM) content of at least 10 wt.-% is accomplished. Preferred concentration ranges which are realized by the concentration step are 10 to 40 wt.-% TDM, in particular 15 to 25 wt.-% TDM.

After addition of the cryoprotectants according to step (b) the suspension is preferably thoroughly mixed, preferably by stirring, to obtain a homogeneous suspension.

Drying of the suspension in step (c) can be carried out by conventional drying processes, in particular by methods such as spray drying, tray drying, fluidized-bed drying, drum drying, or evaporation, optionally after an initial concentration step by e.g. centrifugation. In a preferred embodiment of the invention, drying of the suspension is carried out by freeze-drying, optionally after an initial concentration step. The resulting dry products optionally may be further processed, such as by milling or granulation, to achieve a specific particle size or physical format, in particular before employing them in subsequent step (d).

Mixing of the dried suspension with a further carrier according to step (d) is preferably carried out by using conventional mixers and/or blenders like paddle mixers, screw blenders or drum blenders to obtain a homogeneous product.

"Maltodextrins" are water-soluble carbohydrate mixtures which are obtainable by hydrolysis of starch. They are a mixture of monomers, dimers, oligomers and polymers of glucose. The percentage composition differs depending on the degree of hydrolysis. The degree of hydrolysis is given in dextrose equivalents (DE). Starch has a DE value of 1, glucose has a DE value of 100. Maltodextrins have a DE value of 3 to 20. According to the invention, maltodextrins with any DE values can be used, but preference is given to using maltodextrins with a DE value of 10 to 20.

"Protein hydrolysates" according to the invention are in general mixtures of peptides and amino acids which are preferably obtained by hydrolysis of proteins, wherein the proteins can in particular be of animal, plant or microbial origin. Hydrolysis of the protein can in particular be carried out enzymatically, for example by using the enzymes pepsin or trypsin, or by using acids.

Protein hydrolysates of animal or plant origin are also known as "peptones". Peptones which are obtained by using trypsin are commonly also known as "tryptones". Protein hydrolysates and peptones of animal nature can for example be of bovine or porcine origin. Protein hydrolysates and peptones of animal origin can for example be obtained by hydrolyzing heart or meat proteins, milk, casein or albumin. An example of a preferred protein hydrolysate of plant origin is soy peptone. According to the invention, peptones of plant origin are particularly preferred, above all soy peptone.

Preferred protein hydrolysates of microbial origin are yeast extracts. Yeast extracts are obtained by lysis of yeast cells and subsequent removal of the cell walls. Like peptones they contain in particular peptides and amino acids, but besides that they in particular contain also vitamins of the B group in higher amounts. Besides soy peptone, yeast extract is also a particularly preferred protein hydrolysate according to the invention.

The feed additives as mentioned before, like the microbial preparations itself, can be used for the preparation of feed and pharmaceutical compositions and can be added to drinking and rearing water. In a preferred embodiment 50 to 1000 grams of the feed additive, in particular 100 to 500 grams of the feed additive, are used per ton of feed, drinking or rearing water to provide compositions which can be used for feeding animals. The feed and food compositions can be prepared by mixing the feed additive with typical feed or food ingredients, respectively.

A further subject of the present invention is therefore also a feed or food composition, containing a microbial preparation and/or a feed additive according to the invention and preferably at least one further feed or food ingredient.

Thus, a further subject of the present invention is also the use of a microbial preparation of the invention and/or of a feed additive of the invention for preparing a feed or food composition.

Thus, a further subject of the present invention is also a method of preparing a feed or food composition according to the invention, wherein a microbial preparation of the invention and/or a feed additive according to the invention are mixed with further feed or food additives.

A further subject of the present invention is also a pharmaceutical composition containing a microbial preparation according to the invention and at least on pharmaceutically acceptable carrier.

Thus, a further subject of the invention is also the use of a microbial preparation of the invention and/or of a feed additive of the invention for preparing a pharmaceutical composition, in particular a pharmaceutical composition for treating, preventing or mitigating the course of a gut disease.

Thus, a further subject of the present invention is also a method of preparing a pharmaceutical composition according to the invention, wherein a microbial preparation of the invention and/or a feed additive according to the invention are mixed with a pharmaceutically acceptable carrier.

The microorganisms which are contained in the microbial preparations according to the invention may be any kinds of microorganisms, in particular endospore-forming bacteria and/or nonsporulating bacteria. As in particular cells which are nonsporulating need special protection, the microorganisms according to the invention are preferably nonsporulating.

Endospore-forming bacteria which can be contained in the microbial preparations according to the invention may in particular be selected from the genus Bacillus and are more preferably selected from the species *B. subtilis*, *B. amyloliquefaciens*, *B. velezensis*, *B. licheniformis*, *B. paralicheniformis*, *B. pumilus*, *B. megaterium*, *B. lentus*, *B. laterosporus*, *B. alevi*, *B. cereus*, *B. badius*, *B. thurigiensis*, *B. coagulans*, *B. siamensis*, *B. glycinifermentans*, *B. methylotrophicus*, *B. thuringensis*, *B. polyfermenticus*, *B. vallismortis*, *B. tequilensis*, *B. atrophaeus*, *B. mojavensis*, *B. sonorensis*, *B. inaquosus* and *B. safensis*, most preferably they are of the species *Bacillus subtilis, Bacillus velezensis, Bacillus amyloliquefaciens, Bacillus pumilus* or *Bacillus megaterium.*

But as endospore-forming bacteria due to its ability to form endospores do not necessarily need a protecting carrier. Thus, according to the invention more preferably the microbial preparations contain nonsporulating bacteria.

According to the invention the bacteria which are contained in the microbial preparations according to the invention are preferably lactic acid producing bacteria, more preferably of the order Lactobacillales, very preferably of the families Aerococcaceae, Carnobacteriaceae, Enterococcaceae, Lactobacillaceae, Leuconotocaceae or Streptococcaceae, or of the order Bifidobacteriales, very preferably of the family Bifidobacteriaceae, in particular of the genus Bifidobacterium.

In a very preferred embodiment of the invention, the microbial preparations according to the invention contain lactic acid producing bacteria of the family Enterococcaceae, more preferably of the genus Enterococcus, above all of the species Enterococcus faecium, in particular the strain Enterococcus faecium CECT 4515.

Lactic acid producing bacteria, in particular of the family Enterococcaceae, above all the strain Enterococcus faecium CECT 4515, are generally known to have a beneficial effect on the gut of animals and human beings.

Thus, the microbial preparations of the invention, when administered to animals, preferably enhance the health of such animals and/or improve the general physical condition of such animals and/or improve the feed conversion rate of such animals and/or decrease the mortality rate of such animals and/or increase the survival rate of such animals and/or improve the weight gain of such animals and/or increase the productivity of such animals and/or increase the disease resistance of such animals and/or modulate the immune response of such animals and/or establish or maintain a healthy gut microflora in such animals and/or improve the meat quality of such animals, in particular the meat elasticity and/or the meat hardness, and/or reduce the shedding of bacterial and/or viral pathogens through the feces of such animals. In particular, the microbial preparations and compositions of the invention might be used to assist in re-establishing a healthy balance of the gut microflora after administration of antibiotics for therapeutic purposes.

A further subject of the invention is therefore a method of enhancing the health of animals and/or of improving the general physical condition of animals and/or of improving the feed conversion rate of animals and/or of decreasing the mortality rate of animals and/or of increasing the survival rates of animals and/or of improving the weight gain of animals and/or of increasing the productivity of animals and/or of increasing the disease resistance of animals and/or of modulating the immune response of animals and/or of establishing or maintaining a healthy gut microflora in animals and/or of improving the meat quality of animals and/or of reducing the shedding of bacterial and/or viral pathogens through the feces of animals, wherein at least one microbial preparation and/or at least one feed additive and/or at least one composition of the invention is administered to animals.

A further subject of the invention is therefore also a method of enhancing the health of human beings and/or of improving the general physical condition of human beings and/or of increasing the disease resistance of human beings and/or of modulating the immune response of human beings and/or of establishing or maintaining a healthy gut microflora in human beings, wherein at least one microbial preparation and/or at least one composition according to the invention is administered to human beings.

A further subject of the invention is therefore also the use of at least one microbial preparation and/or at least one feed additive and/or at least one composition of the invention for (preparing a composition for) enhancing the health of animals and/or for improving the general physical condition of animals and/or for improving the feed conversion rate of animals and/or for decreasing the mortality rate of animals and/or for increasing the survival rates of animals and/or for improving the weight gain of animals and/or for increasing the productivity of animals and/or for increasing the disease resistance of animals and/or for modulating the immune response of animals and/or for establishing or maintaining a healthy gut microflora in animals and/or for improving the meat quality of animals and/or for reducing the shedding of bacterial and/or viral pathogens through the feces of animals, wherein the at least one microbial preparation and/or at least one composition of the invention is administered to animals.

A further subject of the invention is therefore also the use of at least one microbial preparation and/or at least one composition of the invention for (preparing a composition for) enhancing the health of human beings and/or for improving the general physical condition of human beings and/or for increasing the disease resistance of human beings and/or for modulating the immune response of human beings and/or for establishing or maintaining a healthy gut microflora in human beings, wherein the at least one microbial preparation and/or at least one composition of the invention, is administered to human beings.

A further subject of the invention is therefore also at least one microbial preparation and/or at least one feed additive and/or at least one composition of the invention, for enhancing the health of animals and/or human beings and/or for improving the general physical condition of animals and/or human beings and/or for improving the feed conversion rate of animals and/or for decreasing the mortality rate of animals and/or for increasing the survival rate of animals and/or for improving the weight gain of animals and/or for increasing the productivity of animals and/or for increasing the disease resistance of animals and/or human beings and/or for modulating the immune response of animals and/or human beings and/or for establishing or maintaining a healthy gut microflora in animals and/or human beings and/or for improving the meat quality of animals and/or for reducing the shedding of bacterial and/or viral pathogens through the feces of animals.

"Increasing the productivity of animals" refers in particular to any of the following: production of more or higher quality eggs, milk or meat or increased number of offspring or improved survival of offspring.

By using the microbial preparations, feed additives and compositions of the invention preferably an improvement of at least one of the features as mentioned before is realized, wherein realization of the feature preferably means an improvement of at least 1 %, more preferably of at least 3 or at least 5 %, in comparison to an adequate negative control. As negative control averages known in the animal husbandry field may be used, but preferably as negative control animals which are subjected to the same treatment like the animals tested are used, but without administration of the microbial preparations and/or compositions of the invention.

The methods and uses of the microbial preparations, feed additives and compositions of the invention can be pharmaceutical or non-pharmaceutical, in particular therapeutic or non-therapeutic. In a particularly preferred embodiment of the invention, the methods and uses are non-pharmaceutical, in particular non-therapeutic, preferably feed and food applications.

Preferred food compositions according to the invention are fermented foods and/or dairy products, in particular yoghurt, cheese, milk, butter, curd and natto. The microorganisms according to the invention may in particular be used in beverages and in milk replacers, for examples in a baby formula.

The pharmaceutical compositions according to the invention preferably contain at least one pharmaceutically acceptable carrier. They may further contain other ingredients like typical food ingredients as listed further below.

The pharmaceutical composition according to the invention is preferably a liquid, a paste, an aerosol, a suppository or a dried composition, in particular to be administered to the pharyngeal/respiratory tract, to the gastro-intestinal tract, to the urogenital tract or to the skin. Administration might be carried out by using a medical device, for example a spray, a pump or an inhalator.

Thus, a further subject of the present invention is also a method of preparing a pharmaceutical composition, wherein microbial preparations according to the invention are mixed with a pharmaceutically acceptable carrier and optionally further compounds and wherein the pharmaceutical composition may be provided in the form of a medical device.

A further subject of the present invention is in particular also a nutritional supplement containing microbial preparations according to the invention and at least one pharmaceutically acceptable carrier.

In a preferred embodiment of the invention, the microbial preparations and compositions of the present invention are administered orally to animals or human beings. The administration is preferably carried out in form of feed and food compositions, but may also be carried out by applying the microbial preparations or feed additives of the invention to drinking or rearing water. Thus, a further subject of the present invention is water, in particular an aqueous solution or an aqueous suspension, containing at least one microbial composition or feed additive according to the invention.

The feed, food and pharmaceutical compositions of the invention as well as the drinking water, rearing water, effluent water or wastewater, preferably comprise microorganisms at a rate of about $1 \times 10^2$ to about $1 \times 10^{10}$ CFU/g feed or ml water, preferably in an amount of about $1 \times 10^4$ to about $1 \times 10^9$ CFU/g feed or ml water, more preferably in an amount of $1 \times 10^6$ to $1 \times 10^8$ CFU/g feed or ml water.

Correspondingly, preferred amounts of feed additive of the invention in the feed, food and water compositions of the invention range from 0.01 wt.-% to 10 wt.-%, more preferably from 0.05 wt.-% to 5 wt.-%, in particular from 0.1 wt.-% to 2 wt.-%.

The feed, food and pharmaceutical compositions of the invention may comprise additional carriers or further typical feed ingredients or combinations thereof.

Suitable carriers for the preparation of feed, food and pharmaceutical compositions are the same as mentioned before for the preparation of the feed additive starting from the microbial preparation of the invention.

Suitable typical animal feed ingredients which may be contained in the compositions according to the invention and/or used in the preparation of feed compositions starting from the microbial preparation and/or feed additive according to the invention include one or more of the following: proteins, carbohydrates, fats, further probiotics, prebiotics, enzymes, vitamins, immune modulators, milk replacers, minerals, amino acids, carriers, coccidiostats, acid-based products and/or medicines, such as antibiotics.

Carbohydrates containing components which may be used according to the invention are for example forage, roughage, wheat meal, corn meal, sunflower meal or soya meal, and mixtures thereof.

Proteins containing components which may be used according to the invention are for example soya protein, pea protein, wheat gluten, corn gluten, rice, canola meal, meal of marine animals, in particular fishmeal, meal of terrestrial animals, and mixtures thereof. "Meal of marine animals" includes meat meal, meat and bone meal, blood meal, liver meal, poultry meal, silkworm meal, silkworm pupae meal and combinations thereof.

Fats are typically provided as oils of marine animals, vegetable oils or oils of microorganisms, in particular oils of microalgae, or combinations thereof. Examples of vegetable oils are soybean oil, rapeseed oil, sunflower seed oil, canola oil, cottonseed oil, flaxseed oil and palm oil. Oils of marine animals include fish oil as well as oil of krill, bivalves, squids or shrimps and further fatty oils from fish of the families Engraulidae, Carangidae, Clupeidae, Osmeridae, Scombridae and/or Ammodytidae. Examples of oils of microalgae are in particular oil of Labyrinthulea, preferably oil of Schizochytria or Thraustochytria. Besides the isolated oils the defatted biomass itself may also be used as fat source, i.e. in particular the meal of a marine animals, preferably fishmeal, or a plant meal, in particular soybean meal, rapeseed meal, sunflower meal, canola meal, cottonseed meal and/or flax seed meal.

Proteins containing components which additionally contain fats which may be used according to the invention are for example fish meal, krill meal, bivalve meal, squid meal or shrimp shells, as well as combinations thereof.

The feedstuff according to the invention has preferably a total protein content of 20 to 50 wt.-% and/or a total fat content of 1 to 15 wt.-% and/or a total carbohydrate content of 20 to 60 wt.-%.

Further probiotics (DFM) and/or microorganisms which may be used according to the invention in combination with the microbial preparations and feed additives of the invention are preferably bacteria selected from the species *Bacillus subtilis*, *Bacillus licheniformis*, *Bacillus paralicheniformis*, *Bacillus lentus*, *Bacillus pumilus*, *Bacillus laterosporus*, *Bacillus coagulans*, *Bacillus alevi*, *Bacillus cereus*, *Bacillus badius*, *Bacillus thurigiensis*, *Bacillus amyloliquefaciens*, *Bacillus velezensis*, *Enterococcus faecium*, and *Pediococcus acidilactici*. Preferred examples are *Bacillus pumilus* DSM 32539 and *Bacillus subtilis* DSM 32540 (both deposited with the DSMZ on June 14, 2017 under the provisions of the Budapest Treaty on the International Recognition of the Deposit of Microorganisms for the Purpose of Patent Procedure) and derivatives thereof, *Bacillus licheniformis* DSM 32314 and *Bacillus subtilis* DSM 32315 (both deposited with the DSMZ on May 12, 2016 under the provisions of the Budapest Treaty on the International Recognition of the Deposit of Microorganisms for the Purpose of Patent Procedure) and derivatives thereof, *Bacillus subtilis* PB6 (as described in US Patent No. 7,247,299 and deposited as ATCC Accession No. PTA-6737), which is sold by Kemin under the trademark CLOSTAT®, *Bacillus subtilis* C-3102 (as described in US Patent No. 4,919,936 and deposited as FERM BP- 1096 with the Fermentation Research Institute, Agency of Industrial Science and Technology, in Japan), sold by Calpis as CALSPORIN®, *Bacillus subtilis* DSM 17299, as sold by Chr. Hansen under the trademark GalliPro®, *Bacillus licheniformis* DSM 17236, as sold by Chr. Hansen under the trademark GalliProTect®, a mixture of *Bacillus licheniformis* DSMZ 5749 and *Bacillus subtilis* DSMZ 5750 spores, as sold by Chr. Hansen under the trademark BioPlus®YC, *B. subtilis* DSM 29784, as sold by Adisseo/Novozymes under the trademark Alterion®, *Bacillus subtilis,* as sold by Chr. Hansen under the trademark PORCBOOST®, or *Bacillus coagulans* microorganisms as described in US Patent No. 6,849,256. Other non-Bacillus probiotics, such as *Saccharomyces cerevisiae*, *Pichia pastoris*, *Aspergillus niger*, *Aspergillus oryzae*, or *Hansenula*, may also be used in compositions of the present invention. In particular in food compositions further probiotics which are known to be useful to the human health may be used such as lactic acid producing bacteria, in particular lactobacilli, or Bifidobacteria. If said further probiotics are not formulated as part of the compositions of the present invention, they may be administered together (either at the same time or at different times) with the compositions of the present invention.

Prebiotics which may be used according to the invention are preferably oligosaccharides, in particular selected from galactooligosaccharides, sialyloligosaccharides, lactulose, lactosucrose, fructooligosaccharides, palatinose or isomaltose oligosaccharides, glycosyl sucrose, maltooligosaccharides, isomaltooligosaccharides, cyclodextrins, gentiooligosaccharides, soybean oligosaccharides, xylooligosaccharides, dextrans, pectins, polygalacturonan, rhamnogalacturonan, mannan, hemicellulose, arabinogalactan, arabinan, arabinoxylan, resistant starch, mehbiose, chitosan, agarose, inulin, tagatose, polydextrose, and alginate.

Enzymes which may be used in feed compositions according to the invention and which may aid in the digestion of feed, are preferably selected from phytases (EC 3.1 .3.8 or 3.1.3.26), xylanases (EC 3.2.1.8), galactanases (EC 3.2.1 .89), galactosidases, in particular alpha-galactosidases (EC 3.2.1.22), proteases (EC 3.4), phospholipases, in particular phospholipases A1 (EC 3.1 .1.32), A2 (EC 3.1.1.4), C (EC 3.1.4.3), and D (EC 3.1.4.4), lysophospholipases (EC 3.1 .1.5), amylases, in particular alpha-amylases (EC 3.2.1.1 ); lysozymes (EC 3.2.1 .17), glucanases, in particular beta-glucanases (EC 3.2.1.4 or EC 3.2.1.6), glucoamylases, cellulases, pectinases, or any mixture thereof.

Examples of commercially available phytases include Bio-Feed™ Phytase (Novozymes), Ronozyme® P and HiPhos™ (DSM Nutritional Products), Natuphos™ (BASF), Finase® and Quantum® Blue (AB Enzymes), the Phyzyme® XP (Verenium/DuPont) and Axtra® PHY (DuPont). Other preferred phytases include those described in e.g. WO 98/28408, WO 00/43503, and WO 03/066847.

Examples of commercially available xylanases include Ronozyme® WX and G2 (DSM Nutritional Products), Econase® XT and Barley (AB Vista), Xylathin® (Verenium) and Axtra® XB (Xylanase/beta-glucanase, DuPont). Examples of commercially available proteases include Ronozyme® ProAct (DSM Nutritional Products).

Vitamins which may be used according to the invention are for example vitamin A, vitamin D3, vitamin E, vitamin K, e.g., vitamin K3, vitamin B12, biotin, choline, vitamin B1 , vitamin B2, vitamin B6, niacin, folic acid and pantothenate, e.g. Ca-D-pantothenate, or combinations thereof.

Immmune modulators which may be used are for example antibodies, cytokines, spray-dried plasma, interleukins, or interferons, or combinations thereof.

Minerals which may be used according to the invention are for example boron, cobalt, chloride, chromium, copper, fluoride, iodine, iron, manganese, molybdenum, selenium, zinc, calcium, phosphorous, magnesium, potassium, or sodium, or combinations thereof.

Amino acids which may be used according to the invention are for example lysine, alanine, threonine, methionine or tryptophan, or combinations thereof.

The compositions of the invention may further comprise betaine and/or choline and/or other physiologically effective methyl group donors. The feedstuffs may further comprise polyunsaturated fatty acids, in particular DHA and/or EPA.

Thus, a further embodiment of the invention is also a method of preparing an animal feed composition comprising mixing at least one microbial preparation of the invention, in particular in an amount effective to enhance animal health, in particular gut health, with feed ingredients, such as proteins, lipids and/or carbohydrates, and optionally further beneficial substances, preferably as mentioned before, to provide a feeding product. This method may comprise for example also a pelleting step.

Standard pelleting processes known to those of skill in the art may be used, including extrusion processing of dry or semi-moist feeds. Preferred pelleting temperatures are between about 65° C and about 120° C.

The microbial preparations, feed additives and compositions of the invention can be administered to animals in feed and/or drinking water and/or rearing water over multiple days throughout the animal's life or during particular stages or portions of the animal's life. For example, the microbial preparations and/or compositions can be administered only in a starter diet or only in a finisher diet of farm animals.

The microbial preparations and compositions of the invention can further be employed in a wide dosage range. Daily doses are, for example, in the range of between approximately 1 mg and approximately 500 mg, in particular in the range of approximately 5 mg and approximately 200 mg, in the range of from approximately 10 mg to approximately 100 mg or in the range of from approximately 20 to approximately 60 mg per kilogram live weight.

Preferably according to the invention always an effective amount of the microbial preparations and/or feed additives and/or compositions of the invention is used in the embodiments of the invention. The term "effective amount" refers to an amount which effects at least one beneficial effect to an animal and/or to the environment, in particular with respect to the features as already mentioned before, in comparison to an animal or environment that has not been administered the microbial preparations and/or compositions of the invention, but besides that has been administered the same diet (including feed and other compounds) or the same composition, respectively.

In case of therapeutic applications preferably a therapeutic amount of the microbial preparations and/or feed additives and/or compositions of the invention is used. The term "therapeutic amount" refers to an amount sufficient to ameliorate, reverse or prevent a disease state in an animal. Optimal dosage levels for various animals can easily be determined by those skilled in the art, by evaluating, among other things, the composition's ability to (i) inhibit or reduce pathogenic viruses and/or bacteria in the gastrointestinal tract, in particular in the gut, or in the oral or nasopharyngeal cavity or on the skin or in the gills at various doses, (ii) increase or maintain levels of beneficial bacteria and /or (iii) enhance animal health, in particular gut health, at various doses.

The methods of the present invention may be used for all kind of animals, in particular all kind of vertebrates such as mammals, aquatic animals and birds.

Animals that may benefit from the invention include but are not limited to farm animals, pets, exotic animals, zoo animals, animals used for sports, recreation or work.

In a very preferred embodiment of the invention, the animals are farm animals, which are raised for consumption or as food-producers, such as poultry, swine and ruminants.

The poultry may be selected from productive or domestic poultry, but also from fancy poultry or wild fowl. Preferred productive poultry in this context are chickens, turkeys, ducks and geese. The productive livestock in this context is preferably poultry optimized for producing young stock or poultry optimized for bearing meat. Preferred fancy poultry or wild fowl are peacocks, pheasants, partridges, chukkars, guinea fowl, quails, capercaillies, grouse, pigeons and swans, with quails being especially preferred. Further preferred poultry are ratites, in particular ostriches and emus, as well as parrots.

Ruminants according to the invention are preferably selected from cattle, goat and sheep. In one embodiment, the compositions of this invention may be fed to preruminants to enhance their health and, in particular, to decrease the incidence of diarrhea in these animals. Preruminants are ruminants, including calves, ranging in age from birth to about twelve weeks.

Pets are preferably selected from dogs, cats, domestic birds and domestic exotic animals. Animals used for sports may in particular be horses.

The aquatic animals according to the invention are preferably finfish, in particular of the class Actinopterygii, or crustaceans. Actinopterygii include, in particular, tilapia and other cichlids, carps and other cyprinids, salmons and other salmonids, catfish, in particular African catfish and pangasius, tuna, perch, cod, smelt, milkfish, gourami, seabass, in particular barramundi, seabream, grouper and snakehead fish.

Preferred types of salmon and salmonids in this context are the Atlantic salmon, red salmon (sockeye salmon), masu salmon (cherry salmon), king salmon (Chinook salmon), keta salmon (chum salmon), coho salmon, Danube salmon, Pacific salmon, pink salmon and trout. The aquatic animals according to the invention are very preferred tilapia.

Crustaceans include in particular shrimps, lobster, crabs, prawns and crayfish.

Preferred types of shrimps in this context are *Litopenaeus*, *Farfantepenaeus* and *Penaeus,* in particular *Penaeus stylirostris*, *Penaeus vannamei*, *Penaeus monodon*, *Penaeus chinensis*, *Penaeus occidentalis*, *Penaeus calif orniensis*, *Penaeus semisulcatus*, *Penaeus esculentu*, *Penaeus setiferus*, *Penaeus japonicus*, *Penaeus aztecus*, *Penaeus duorarum*, *Penaeus indicus,* and *Penaeus merguiensis.* Very preferred according to the invention is the shrimp *Penaeus vannamei*, also called whiteleg shrimp.

The aquatic animals, and in particular the shrimp, which are treated or fed with the microorganisms and/or preparations and/or compositions according to the invention can in particular be larvae, post-larvae or juvenile shrimp.

The aquatic animals may in particular also be fish which is subsequently processed into fish meal or fish oil. In this connection, the fish are preferably herring, pollack, cod or small pelagic fish like anchovy, blue whiting, capelin, driftfish, jack, mackerel, menhaden, sardine or scad fish. The fish meal or fish oil thus obtained, in turn, can be used in aquaculture for farming edible fish or crustaceans.

The aquatic animals may further be oysters, clams, cockles, arkshells, bivalves, mussels or scallops.

However, the aquatic animals may also be small organisms which are used as feedstuff in aquaculture. These small organisms may take the form of, for example, nematodes, small crustaceans or rotifers.

The farming of aquatic animals may take place in ponds, tanks, basins or else in segregated areas in the sea or in lakes or in rivers, in particular in this case in cages or net pens. Farming may be used for farming the finished edible fish, but also may be used for farming fry which are subsequently released so as to restock the wild fish stocks.

In salmon farming, the fish are preferably first grown into smolts in freshwater tanks or artificial watercourses and then grown on in cages or net pens which float in the sea, ponds or rivers and which are preferably anchored in bays or fjords.

Accordingly, the feed composition according to the invention is preferably a feedstuff for use in the farming of the above-mentioned animals. Preferred applications for animal feed according to the invention is in the feed of cattle, swine, poultry, fish, crustacean or companion animals, with swine, in particular weaned piglets, and poultry being particularly preferred. Spore forming microorganisms, in particular of the genus *Bacillus,* are particularly suited for this application as they produce heat resistant spores which can be mixed into feed prior to the pelleting process of animal feed. Microorganisms according to the invention are very preferably used in milk replacers for cattle, goats, sheep and swine or as post-pelleting spray-on coating for pelleted feed material.

The microorganisms to be used according to the present invention can be obtained by culturing the microorganisms of the invention according to methods well known in the art, including by using the media and other methods as described for example by Herranz et al. (Applied Microbiology and Biotechnology 56, 378-383 (2001)), Shibata et al. (Enzyme and Microbial Technology 41, 149-155 (2007)) and Sun et al. (Biotechnology Letters 37, 1379-1383 (2015)). Conventional large-scale microbial culture processes include submerged fermentation, solid state fermentation, or liquid surface culture. Fermentation is configured to obtain high levels of colony forming units of the microbial cells. Optionally, the fermentation broth thus obtained may be washed, for example via a diafiltration process, to remove residual fermentation broth and metabolites. Fermentation broths as used for the preparation of microbial preparations according to the invention preferably contain cells in an amount of $5 \times 10^{10}$ to $5 \times 10^{12}$, more preferably in an amount of $1 \times 10^{11}$ to $4 \times 10^{12}$ cfu/g.

Working examples

General:

All cryoprotectants employed were purchased from commercial suppliers such as Brenntag (Germany) or SigmaAldrich (Merck, Germany).

The residual moisture was measured by putting a defined mass of a sample for 3 h at 105 °C into a conventional convection drying oven. The sample was cooled down to room temperature in a desiccator and weighed again. The residual moisture is calculated as follows:

$$\text{Residual moisture} = (\text{weight of sample before drying} - \text{weight of sample after drying}) * 100 / \text{weight of sample before drying [\%]}.$$

From this value, the total dry matter content is calculated as follows:

$$\text{Total dry matter} = 1 - \text{residual moisture [\%]}.$$

The water activity was measured using the device AquaLab 4TE Water Activity Meter (Aqualab, Germany).

The enumeration and differentiation of probiotic *Enterococcus faecium* capable of germinating was conducted according to the VDLUFA 28.2.3 method (Verband deutscher landwirtschaftlicher Untersuchungs- und Forschungsanstalten, Germany). Thus, an initial suspension of the sample is prepared in a diluent such as phosphate buffered saline (PBS), dilutions are prepared with polysorbate peptone salt solution (PPS), spread-plated on Slanetz & Bartley agar with subsequent incubation at 37 °C for 36-48 hours under aerobic conditions. All dark red colonies are counted and the number of colony forming units per gram (CFU/g) is calculated according to the dilution factor. A typical standard error of this method is +-30%.

The blending of the freeze-dried microbial preparation with a carrier such as limestone or dextrose was carried out by addition of a defined amount of the microbial preparation to a defined amount of carrier in order to achieve the targeted CFU/g in the blend. The mixer was subsequently homogenized by using a screw mixer.

Working example 1: Determining the influence of different cryoprotectants on the survival rate of bacteria

To a concentrated fermentation broth containing *Enterococcus faecium* CECT 4515 with a total dry matter content of 20.3 % and a titer of 2.28E11 CFU/g, maltodextrin and optionally a second cryoprotectant as depicted in table 1 were added as powder. The suspension was stirred in order to dissolve the cryoprotectant and to homogenize the mixture. After 30 minutes of stirring, the obtained material was filled in stainless steel trays with a filling height of 3 cm. The trays were freeze-dried and the residual moisture content, water activity as well as the titer of the biomass were measured (Table 2). The obtained biomass was stored at 20°C for 6 and 10 weeks and titers were measured accordingly after each point in time. In order to evaluate the stability upon freeze-drying as well as storage, survival rates for each point in time were calculated.

As can be learnt from table 2, survival rates of cells which were freeze-dried by using a combination of maltodextrin with either soy peptone or yeast extract as cryoprotectants were significantly higher than either using maltodextrin as only carrier or using maltodextrin with an alternative further cryoprotectant.

Working example 2: Determining the influence of the amount and distribution of selected cryoprotectants maltodextrin, yeast extract and soy peptone on the survival rates of bacteria

To a concentrated fermentation broth containing *Enterococcus faecium* CECT 4515 with a total dry matter content of 18.5 % and a titer of 2.35E11 CFU/g, maltodextrin, yeast extract, and soy peptone were added as powder in amounts as depicted in table 3. The suspension was stirred in order to dissolve the cryoprotectants and to homogenize the mixture. After 30 minutes of stirring, the obtained material was filled in stainless steel trays with a filling height of 3 cm. The trays were freeze-dried and the residual moisture content and the titer of the biomass were measured. The obtained microbial preparations were stored at 40 °C for an accelerated evaluation of the storage stability for 4, 8, and 16 weeks and titers were measured accordingly after each point in time. In order to evaluate the stability upon storage, survival rates for each point in time were calculated.

As can be learnt from table 3, survival rates of cells which were freeze-dried by using a combination of maltodextrin with either soy peptone or yeast extract as cryoprotectants were significantly higher than by using either only yeast extract or a mixture of yeast extract and soy peptone as cryoprotectants.

Working example 3: Determining the survival rates of bacteria in blended products

To a concentrated fermentation broth containing *Enterococcus faecium* CECT 4515, maltodextrin and yeast extract or maltodextrin and soy peptone were added as powder in amounts relative to the total dry matter content in the fermentation broths according to Exp. 13-16 as disclosed in table 3. The suspension was stirred in order to dissolve the cryoprotectants and to homogenize the mixture. After 30 minutes of stirring the obtained material was filled into stainless-steel trays with a filling height of 3 cm. The trays were freeze-dried and the titer of the biomass was measured (Table 4). The obtained microbial preparations were stored at 5 °C for an evaluation of the storage stability for 1, 2, and 3 months.

The four microbial preparations obtained by this procedure were used to produce blended products mixed to a titer of 1-3*1 E10 CFU/g or 1-3*1 E9 CFU/g with either limestone or dextrose as carrier and stored at 20°C for an evaluation of the storage stability for 1, 2, and 3 months according to Table 4.

As can be learnt from table 4, the survival rates of the bacteria as contained in the microbial preparations were still very good, after blending the microbial preparations with typical carriers like limestone and dextrose.

Table 1: Preparation of the broth with differing cryoprotectants for subsequent freeze-drying.

| Exp. # | mass concentrated fermentation broth [g] | Total dry matter broth before addition of cryoprotectants [g] | mass malto-dextrin [g] | mass 2nd additive [g] | 2nd additive | Total dry matter in broth after addition of cryoprotectants | part of the invention |
|---|---|---|---|---|---|---|---|
| 1 | 120 | 24 | 0 | 0 | - | 20% | no |
| 2 | 120 | 24 | 12 | 0 | - | 28% | no |
| 3 | 120 | 24 | 12 | 6 | Dextrose | 32% | no |
| 4 | 120 | 24 | 12 | 6 | Sucrose | 31% | no |
| 5 | 120 | 24 | 12 | 6 | Glycerol | 30% | no |
| 6 | 120 | 24 | 12 | 6 | $CaCO_3$ | 30% | no |
| 7 | 120 | 24 | 12 | 6 | Corn Starch | 29% | no |
| 8 | 120 | 24 | 12 | 6 | Methylcellulose | 30% | no |
| 9 | 120 | 24 | 12 | 6 | Hydroxypropylmethylcellulose | 32% | no |
| 10 | 120 | 24 | 12 | 6 | Soy peptone | 30% | yes |
| 11 | 120 | 24 | 12 | 6 | Yeast extract | 31% | yes |

Table 2: Analysis of the survival rates of bacteria in the different freeze-dried microbial preparations

| Exp. # | Residual moisture | water activity | Titer after freeze drying [CFU/g] | Titer after 6 weeks at 20°C [CFU/g] | Titer after 10 weeks at 20°C [CFU/g] | survival rate after freeze drying | survival rate after 6 weeks at 20°C | survival rate after 10 weeks at 20°C | Part of the invention |
|---|---|---|---|---|---|---|---|---|---|
| 1 | 1,9% | 0,48 | 2,6E+12 | 2,7E+10 | <1E+03 | 130% | 1,3% | 0,0% | no |
| 2 | 3,8% | 0,32 | 5,9E+11 | 3,6E+10 | 1,9E+09 | 49% | 2,9% | 0,2% | no |
| 3 | 2,1% | 0,47 | 9,0E+11 | 0,0E+00 | 0,0E+00 | 89% | 0,0% | 0,0% | no |
| 4 | 1,9% | 0,44 | 7,5E+11 | 7,0E+10 | 3,3E+09 | 58% | 5,4% | 0,3% | no |
| 5 | 1,2% | 0,24 | 1,0E+12 | 2,1 E+09 | 2,2E+08 | 125% | 0,3% | 0,0% | no |
| 6 | 3,2% | 0,12 | 9,0E+11 | 4,1E+10 | 4,2E+10 | 60% | 2,7% | 2,8% | no |
| 7 | 2,7% | 0,23 | 1,0E+12 | 5,4E+10 | 3,1E+10 | 89% | 4,8% | 2,7% | no |
| 8 | 2,2% | 0,22 | 8,3E+11 | 7,6E+09 | 4,9E+09 | 71% | 0,7% | 0,4% | no |
| 9 | 2,4% | 0,22 | 6,5E+11 | 1,1E+10 | 9,0E+09 | 54% | 0,9% | 0,7% | no |
| 10 | 1,8% | 0,22 | 1,3E+12 | 3,34E+11 | 3,3E+11 | 114% | 30,1% | 29,8% | Yes |
| 11 | 1,8% | 0,29 | 9,2E+11 | 3,5E+11 | 2,6E+11 | 68% | 26,0% | 19,3% | Yes |

Table 3: Determining the influence of maltodextrin, yeast extract and soy peptone on the survival rates of bacteria

| Exp. # | Mass concentrated fermentation broth [g] | Mass maltodextrin [g] | Mass yeast extract [g] | Mass soy peptone [g] | Residual moisture after drying [%] | Titer after drying [CFU/g] | Titer after 4 weeks storage at 40°C [CFU/g] | Titer after 8 weeks storage at 40°C [CFU/g] | Titer after 16 weeks storage at 40°C [CFU/g] | survival rate after 4 weeks at 40°C | survival rate after 8 weeks at 40°C | survival rate after 16 weeks at 40°C | Part of the invention |
|---|---|---|---|---|---|---|---|---|---|---|---|---|---|
| 12 | 2000 | 0 | 200 | 0 | 7,0 | 9,25E+11 | 1,01E+11 | 7,83E+09 | <1E+03 | 11% | 1% | 0% | No |
| 13 | 2000 | 200 | 50 | 0 | 3,8 | 9,46E+11 | 6,33E+11 | 5,30E+11 | 4E+11 | 67% | 56% | 39% | Yes |
| 14 | 2000 | 200 | 100 | 0 | 6,7 | 8,82E+11 | 5,00E+11 | 4E+11 | 2E+11 | 57% | 40% | 20% | Yes |
| 15 | 2000 | 200 | 100 | 0 | 6,3 | 7,60E+11 | 4,23E+11 | 3,20E+11 | 1E+11 | 56% | 42% | 17% | Yes |
| 16 | 2000 | 200 | 0 | 50 | 4,0 | 8,59E+11 | 5,53E+11 | 4,18E+11 | 2E+11 | 64% | 49% | 24% | Yes |
| 17 | 2000 | 200 | 0 | 100 | 6,2 | 8,51E+11 | 5,52E+11 | 4E+11 | 4E+11 | 65% | 48% | 44% | Yes |
| 18 | 2000 | 400 | 0 | 200 | 6,6 | 6,33E+11 | 4,30E+11 | 1,49E+11 | 7E+08 | 68% | 23% | 0% | Yes |
| 19 | 2000 | 0 | 300 | 100 | 5,6 | 7,59E+11 | 9,97E+10 | 1,32E+10 | <1E+03 | 13% | 2% | 0% | No |

Table 4: Survival rate of bacteria in blended products (MD = maltodextrin, YE = yeast extract, SP = soy peptone. Ratios of individual cryoprotectants in employed biomass according to Exp. 13-16)

| Exp. # | Description | Content biomass | Content carrier | Water activity | Initial titer [CFU/g] | Titer after 1 month [CFU/g] | Titer after 2 months [CFU/g] | Titer after 3 months [CFU/g] | survival rate after 1 month | survival rate after 2 months | survival rate after 3 months | Part of the invention |
|---|---|---|---|---|---|---|---|---|---|---|---|---|
| 20 | Biomass MD: YE 2:1 | 100% | 0% | 0,33 | 5,6E+11 | 6,0E+11 | 5,8E+11 | 6,4E+11 | 107% | 107% | 114% | Yes |
| 21 | Biomass MD: YE 2:1 blended with limestone | 3,9% | 96,1% | 0,31 | 2,2E10 | 1,9E+10 | 1,9E+10 | 2,0E+10 | 86% | 86% | 91% | Yes |
| 22 | Biomass MD: YE 2:1 blended with limestone | 0,34% | 99,66% | 0,27 | 1,9E+09 | 1,8E+09 | 1,4E+09 | 1,8E+09 | 95% | 74% | 95% | Yes |
| 23 | Biomass MD: YE 2:1 blended with dextrose | 2,9% | 97,1% | 0,36 | 1,6E+10 | 1,5E+10 | 1,3E+10 | 1,3E+10 | 94% | 81% | 81% | Yes |
| 24 | Biomass MD: YE 4:1 blended with limestone | 2,1% | 97,9% | 0,42 | 2,0E+10 | 2,0E+10 | 2,1E+10 | 1,4E+10 | 100% | 105% | 70% | Yes |
| 25 | Biomass MD: SP 4:1 blended with limestone | 2,2% | 97,8% | 0,33 | 1,9E+10 | 1,4E+10 | 1,5E+10 | 1,4E+10 | 74% | 79% | 74% | Yes |

**Claims**

1. Microbial preparations containing maltodextrin and at least one protein hydrolysate.

2. Microbial preparations according to claim 1, wherein the at least one protein hydrolysate is selected from peptones, in particular soy peptone, and yeast extract and mixtures thereof.

3. Microbial preparations according to claim 1 or 2, containing 15 to 45 wt.-%, preferably 20 to 40 wt.-%, in particular 25 to 35 wt.-%, maltodextrin and 6 to 24 wt.-%, preferably 10 to 20 wt.-%, in particular 12 to 18 wt.-% of at least one protein hydrolysate, in particular a peptone, preferably soy peptone, or a yeast extract.

4. Microbial preparations according to any of the preceding claims, containing 40 to 75 wt.-%, preferably 45 to 70 wt.-%, in particular 50 to 65 wt.-% of a dried bacterial fermentation broth.

5. Microbial preparations according to any of claims 1 to 4, containing lactic acid producing bacteria, preferably of the order Lactobacillales, very preferably of the families Aerococcaceae, Carnobacteriaceae, Enterococcaceae, Lactobacillaceae, Leuconotocaceae or Streptococcaceae, or of the order Bifidobacteriales, very preferably of the family Bifidobacteriaceae, in particular of the genus Bifidobacterium.

6. Microbial preparations according to claim 5, wherein the lactic acid producing bacteria of the family Enterococcaceae are of the genus Enterococcus, preferably of the species *Enterococcus faecium,* more preferably of the strain *Enterococcus faecium* CECT 4515.

7. Feed additive containing a microbial preparation according to any of the preceding claims, preferably in an amount of 0.1 to 10 wt.-%, more preferably in an amount of 0.2 to 5 wt.-%, and a carrier, preferably selected from limestone, dextrose, maltodextrin and combinations thereof, wherein the feed additive preferably contains microorganisms in an amount of $1 \times 10^9$ to $1 \times 10^{11}$, in particular $5 \times 10^9$ to $5 \times 10^{10}$, cfu per g feed additive.

8. Method of preparing a feed additive according to claim 7, wherein a microbial biomass is mixed with maltodextrin and at least one protein hydrolysate, preferably a peptone, in particular soy peptone, or a yeast extract, and the microbial preparation thus obtained is subsequently mixed with a carrier to obtain the feed additive.

9. Method according to claim 8 comprising the following steps:

   a) Preparation of a bacterial fermentation broth by cultivating bacteria in a fermentation medium,
   b) Addition of maltodextrin and at least one protein hydrolysate to the fermentation broth,
   c) Drying of the suspension thus obtained,
   d) Mixing of the dried suspension with a further carrier, preferably selected from limestone, dextrose and mixtures thereof.

10. Feed or food composition containing a microbial preparation according to any of claims 1 to 6 or a feed additive according to claim 7 and preferably at least one further feed or food ingredient selected from carriers, proteins, carbohydrates, fats, further probiotics, prebiotics, enzymes, vitamins, immune modulators, milk replacers, minerals, amino acids, carriers, coccidiostats, acid-based products and/or medicines, such as antibiotics.

11. Pharmaceutical composition containing a microbial preparation according to any of claims 1 to 6 or a feed additive according to claim 7 and at least one pharmaceutically acceptable carrier.

12. A method of feeding animals, wherein the animals are fed with at least one microbial preparation according to any of claims 1 to 6 and/or with a feed additive according to claim 7 and/or with a feed or food composition according to claim 10 and/or with a pharmaceutical composition according to claim 11, wherein the animals are preferably selected from mammals, in particular swine, preferably weaned piglets, ruminants, birds, in particular chickens, and aquatic animals.

13. Method of treating and/or preventing and/or mitigating the course of a disease, preferably a gut disease, wherein at least one preparation according to any of claims 1 to 6 and/or a feed additive according to claim 7 and/or a feed or food composition according to claim 10 and/or a pharmaceutical composition according to claim 11 is administered to animals in need thereof.

**14.** Use of a microbial preparation according to any of claims 1 to 6 and/or of a feed additive according to claim 7 for the manufacture of a feedstuff and/or a foodstuff and/or a pharmaceutical composition, in particular a pharmaceutical composition for the treatment or prevention or mitigation of the course of a gut disease.

**15.** Use of a microbial preparation according to any of claims 1 to 6 and/or of a feed additive according to claim 7 and/or of a feed or food composition according to claim 10 for preparing a composition for enhancing the health of animals and/or for improving the general physical condition of animals and/or for improving the feed conversion rate of animals and/or for decreasing the mortality rate of animals and/or for increasing the survival rates of animals and/or for improving the weight gain of animals and/or for increasing the productivity of animals and/or for increasing the disease resistance of animals and/or for modulating the immune response of animals and/or for establishing or maintaining a healthy gut microflora in animals and/or for improving the meat quality of animals and/or for reducing the shedding of bacterial and/or viral pathogens through the feces of animals.

Europäisches
Patentamt

European
Patent Office

Office européen
des brevets

# EUROPEAN SEARCH REPORT

**Application Number**

EP 21 20 2048

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/217368 A1 (PRAKASH SATYA [CA] ET AL) 8 September 2011 (2011-09-08) * paragraphs [0027] – [0030], [0109] – [0182]; claims 1-25 * | 1-15 | INV. A23K10/18 A23L33/135 A61K35/744 A61K35/745 A61K35/747 C12N1/04 C12N1/20 |
| X | US 2007/254353 A1 (STAVNSBJERG RIKKE [DK] ET AL) 1 November 2007 (2007-11-01) * paragraphs [0023], [0075], [0079] – [0081]; claim 14; examples 1-4 * | 1-15 | |
| X | US 2020/224151 A1 (DROUILLARD JAMES SCOTT [US] ET AL) 16 July 2020 (2020-07-16) * paragraphs [0020] – [0031], [0100], [0106], [0258]; figures 5,6,8-10,28; examples 1-20 * | 1-15 | |
| X | US 2011/189147 A1 (GARNER MATTHEW RYAN [US] ET AL) 4 August 2011 (2011-08-04) * paragraph [0119]; examples 1,10 * | 1-15 | |
| A | US 2021/077545 A1 (REHBERGER THOMAS [US] ET AL) 18 March 2021 (2021-03-18) * the whole document * | 1-15 | TECHNICAL FIELDS SEARCHED (IPC) A23K C11C A23L C12R A61K C12N |
| A | WO 2020/144207 A1 (EVONIK OPERATIONS GMBH [DE]) 16 July 2020 (2020-07-16) * claims 1-17 * | 1-15 | |
| A | EP 3 447 122 A1 (EVONIK DEGUSSA GMBH [DE]) 27 February 2019 (2019-02-27) * paragraphs [0077], [0078]; examples 3-5 * | 1-15 | |
| A | WO 2020/225020 A1 (EVONIK OPERATIONS GMBH [DE]) 12 November 2020 (2020-11-12) * page 5, lines 29-30 * * page 8, lines 14-15 * | 6 | |

The present search report has been drawn up for all claims

| Place of search | Date of completion of the search | Examiner |
|---|---|---|
| The Hague | 25 March 2022 | De Jong, Ellen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 20 2048

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2022

| Patent document cited in search report | | Publication date | Patent family member(s) | | Publication date |
|---|---|---|---|---|---|
| US 2011217368 | A1 | 08-09-2011 | AU | 2010242498 A1 | 22-12-2011 |
| | | | BR | PI1009920 A2 | 15-03-2016 |
| | | | CA | 2796929 A1 | 04-11-2010 |
| | | | CN | 102481322 A | 30-05-2012 |
| | | | DK | 2419114 T3 | 25-07-2016 |
| | | | EP | 2419114 A1 | 22-02-2012 |
| | | | EP | 3067056 A1 | 14-09-2016 |
| | | | ES | 2578081 T3 | 20-07-2016 |
| | | | HR | P20160598 T1 | 29-07-2016 |
| | | | JP | 6100526 B2 | 22-03-2017 |
| | | | JP | 2012525338 A | 22-10-2012 |
| | | | JP | 2015127338 A | 09-07-2015 |
| | | | KR | 20120015335 A | 21-02-2012 |
| | | | PL | 2419114 T3 | 30-09-2016 |
| | | | RU | 2011148944 A | 10-06-2013 |
| | | | US | 2011217368 A1 | 08-09-2011 |
| | | | WO | 2010124387 A1 | 04-11-2010 |
| US 2007254353 | A1 | 01-11-2007 | AT | 466070 T | 15-05-2010 |
| | | | AU | 2005215858 A1 | 01-09-2005 |
| | | | BR | PI0507956 A | 17-07-2007 |
| | | | CA | 2557496 A1 | 01-09-2005 |
| | | | CN | 1922305 A | 28-02-2007 |
| | | | DK | 1720974 T3 | 09-08-2010 |
| | | | EA | 200601540 A1 | 29-12-2006 |
| | | | EP | 1720974 A1 | 15-11-2006 |
| | | | EP | 2223609 A1 | 01-09-2010 |
| | | | ES | 2344415 T3 | 26-08-2010 |
| | | | JP | 5727308 B2 | 03-06-2015 |
| | | | JP | 2007522809 A | 16-08-2007 |
| | | | JP | 2011234725 A | 24-11-2011 |
| | | | KR | 20060131854 A | 20-12-2006 |
| | | | MA | 28484 B1 | 01-03-2007 |
| | | | NZ | 549600 A | 26-03-2010 |
| | | | PL | 1720974 T3 | 29-10-2010 |
| | | | US | 2007254353 A1 | 01-11-2007 |
| | | | WO | 2005080548 A1 | 01-09-2005 |
| | | | ZA | 200606222 B | 30-01-2008 |
| US 2020224151 | A1 | 16-07-2020 | AU | 2018215216 A1 | 12-09-2019 |
| | | | BR | 112019015792 A2 | 17-03-2020 |
| | | | CA | 3051249 A1 | 09-08-2018 |
| | | | CL | 2019002135 A1 | 13-12-2019 |
| | | | CN | 110475479 A | 19-11-2019 |
| | | | CO | 2019008826 A2 | 17-01-2020 |
| | | | EP | 3577213 A1 | 11-12-2019 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 21 20 2048

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2022

| Patent document cited in search report | Publication date | Patent family member(s) | Publication date |
|---|---|---|---|
| | | JP 2020508696 A | 26-03-2020 |
| | | KR 20190126309 A | 11-11-2019 |
| | | MA 47439 A | 11-12-2019 |
| | | PH 12019501780 A1 | 29-06-2020 |
| | | SG 11201906748P A | 27-08-2019 |
| | | US 2020224151 A1 | 16-07-2020 |
| | | WO 2018144653 A1 | 09-08-2018 |
| US 2011189147 A1 | 04-08-2011 | US 2011189147 A1 | 04-08-2011 |
| | | US 2013244308 A1 | 19-09-2013 |
| US 2021077545 A1 | 18-03-2021 | BR 112018074133 A2 | 06-03-2019 |
| | | CN 109563472 A | 02-04-2019 |
| | | EP 3464648 A1 | 10-04-2019 |
| | | RU 2018144879 A | 25-06-2020 |
| | | US 2017340682 A1 | 30-11-2017 |
| | | US 2021077545 A1 | 18-03-2021 |
| | | WO 2017205645 A1 | 30-11-2017 |
| WO 2020144207 A1 | 16-07-2020 | BR 112021013484 A2 | 14-09-2021 |
| | | CN 113330107 A | 31-08-2021 |
| | | EP 3908651 A1 | 17-11-2021 |
| | | JP 2022516797 A | 02-03-2022 |
| | | WO 2020144207 A1 | 16-07-2020 |
| EP 3447122 A1 | 27-02-2019 | NONE | |
| WO 2020225020 A1 | 12-11-2020 | AR 118817 A1 | 03-11-2021 |
| | | CN 113825408 A | 21-12-2021 |
| | | EP 3962290 A1 | 09-03-2022 |
| | | TW 202108006 A | 01-03-2021 |
| | | WO 2020225020 A1 | 12-11-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7247299 B
- US 4919936 A
- US 6849256 B
- WO 9828408 A
- WO 0043503 A
- WO 03066847 A

**Non-patent literature cited in the description**

- *Official Publication of American Feed Control Officials,* 2006, ISBN 1-878341-18-9
- **HERRANZ et al.** *Applied Microbiology and Biotechnology,* 2001, vol. 56, 378-383
- **SHIBATA et al.** *Enzyme and Microbial Technology,* 2007, vol. 41, 149-155
- **SUN et al.** *Biotechnology Letters,* 2015, vol. 37, 1379-1383